# EUROPÄISCHE PATENTSCHRIFT

(11) **EP 1 242 376 B1**
(45) Veröffentlichungstag und Bekanntmachung des Hinweises auf die Patenterteilung: **09.06.2004**
(21) Anmeldenummer: 00988767.0
(22) Anmeldetag: 30.11.2000
(51) Int. Cl.: C07D 209/34

(54) **VERFAHREN ZUR HERSTELLUNG VON OXINDOLEN**
METHOD FOR PRODUCING OXINDOLES
PROCEDE DE FABRICATION D'OXINDOLES

(30) Priorität: 27.12.1999 AT 218299
(43) Veröffentlichungstag der Anmeldung: 25.09.2002
(73) Patentinhaber: DSM Fine Chemicals Austria Nfg GmbH & Co KG, 4021 Linz (AT)
(72) Erfinder: HENDEL, Wolfram, A-4060 Leonding (AT); SCHWENDINGER, Karl, A-4040 Linz (AT); FELFER, Ulfried, A-4040 Linz (AT)
(74) Vertreter: Lindinger, Ingrid
(86) Internationale Anmeldenummer: PCT/EP2000/012010
(87) Internationale Veröffentlichungsnummer: WO 2001/047884

(56) Entgegenhaltungen:
- US-A- 5 973 165
- DATABASE HCA [Online] CHEMICAL ABSTRACTS SERVICE, COLUMBUS, OHIO, US; abstract no. 123:285775, XP002167820 & JP 07 196610 A (KURARAY CO) 1. August 1995 (1995-08-01)

## Beschreibung

Oxindole sind wertvolle Zwischenprodukte in der Synthese von pharmazeutischen Produkten, wie etwa von Herzmitteln oder von Tyrosinkinase - Inhibitoren, die das Wachstum der Haut regulieren.

Aus J. Chem. Educ. 1993, 70 (4), S.332 ist bekannt, dass 2-Oxindole durch eine zweistufige Reaktion, bei der im ersten Schritt Isatin mit Hydrazinhydrat in wasserfreiem Methanol zu dem Zwischenprodukt Isatinhydrazon umgesetzt und das Zwischenprodukt isoliert und gereinigt wird. Im zweiten Schritt wird das gereinigte und getrocknete Zwischenprodukt einer Wolf-Kishner Reduktion in einer wasserfreien Ethanollösung in Gegenwart einer starken Base wie Natriumethoxid unterzogen. Die mit dieser Herstellmethode erzielten Ausbeuten liegen bei bis zu etwa 69%.
Der Nachteil dieser Variante ist jedoch die Notwendigkeit, das Zwischenprodukt zu isolieren, zu reinigen und zu trocknen, bevor der Reduktionsschritt erfolgen kann. Ein weiterer Nachteil ist die Verwendung von teurem, wasserfreien Ethanol als Lösungsmittel aufgrund der Reaktionsfreudigkeit des Natriumethoxides mit Wasser.
Eine weitere Herstellungsvariante beschreibt Synth. Commun. 1994, 24 (20), S. 2835-41. Gemäß dieser Variante wird Isatin zuerst in reinem Hydrazin gelöst und dann mit reinem Hydrazin unter Rückflusskochen zu 2-Oxindol in Ausbeuten bis zu 76% umgesetzt.
Diese Methode benötigt jedoch eine große Menge an reinem Hydrazin, das als Lösungsmittel und als Reagens dient. Bei der Verwendung von reinem Hydrazin besteht jedoch bei Wärmezufuhr oder bei Reaktionen mit Oxidierungsmitteln bekanntermaßen Explosionsgefahr, sodass besondere Vorsichtsmaßnahmen getroffen werden müssen.
Als Verbesserung wird in US 5,973,165 vorgeschlagen 2-Oxindole durch Umsetzung von Isatin mit Hydrazinhydrat in Anwesenheit einer schwachen Base als Katalysator und in einem polaren Lösungsmittel herzustellen. Um das gewünschte Endprodukt in reiner Form zu erhalten wird nach Beendigung der Reaktion 2-Oxindol (Reinheit etwa 97%) zuerst extrahiert, das Extrakt getrocknet und anschließend in einem geeigneten Lösungsmittel gelöst, sodann wird der Lösung Aktivkohle zur Entfärbung zugesetzt. Nach dem Abfiltrieren der Aktivkohle wird 2-Oxindol aus der Lösung auskristallisiert und in einer Reinheit von 99,5% erhalten. Die Ausbeuten die mit diesem Verfahren erzielt werden liegen gemäß Beispiel 1 bei 85%, ansonsten jedoch bei 52 bis 72%.

Aufgabe der vorliegenden Erfindung war es ein Verfahren zu finden, das die Herstellung von Oxindolen in hoher Ausbeute und Reinheit ermöglicht, wobei aufwendige Reinigungsschritte vermieden werden.

Gegenstand der Erfindung ist daher ein Verfahren zur Herstellung von 2-Oxindolen der Formel (II) 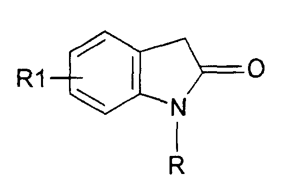 in der R H, CH₃, Phenyl oder Benzyl und R₁ H, C₁-C₄-Alkyl, C₁-C₄-Alkoxy, Phenyl, Phenoxy, Halogen, Amino, Nitro oder Hydroxy sein können, durch Umsetzung der korrespondierenden Isatinen der Formel (I) 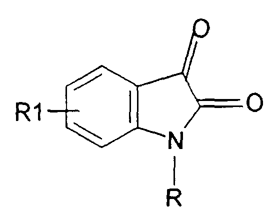 in einem polaren Lösungsmittel mit Hydrazinhydrat in Gegenwart eines tertiären Amins als Katalysator, das dadurch gekennzeichnet ist, dass das Isatin bei einer Temperatur von 15 bis 185°C zu dem entsprechenden Isatinhydrazon umgesetzt wird, das direkt durch Zusatz von Diazabicyclooctan und/oder Diazabicycloundecan und/oder Ethyldiisopropylamin als Katalysator bei Temperaturen von 100 bis 185°C unter Abdestillieren des gebildeten Reaktionswassers zu dem entsprechenden 2-Oxindol der Formel weiterreagiert, welches dann durch Abdestillieren des Lösungsmittels und Kristallisieren aus dem Reaktionsgemisch isoliert wird.

Bei dem erfindungsgemäßen Verfahren wird ein Isatin der Formel (I) zu den korrespondierenden 2-Oxindolen der Formel (II) umgesetzt.
Die als Ausgangsverbindung verwendeten Isatine können dabei am Stickstoffatom durch H, CH₃, Phenyl oder Benzyl substituiert sein. Bevorzugt ist das Stickstoffatom durch H substituiert. Weiters können die Ausgangsverbindungen in Position 4, 5, 6 oder 7 durch H, C₁-C₄-Alkyl, C₁-C₄-Alkoxy, Phenyl, Phenoxy, Halogen, Amino, Nitro oder Hydroxy substituiert sein. Bevorzugte Substituenten sind H oder OCH₃, besonders bevorzugt ist H.
Das entsprechende Isatin wird in einem polaren Lösungsmittel vorgelegt. Geeignete Lösungsmittel sind dabei Alkohole mit einem Siedepunkt ab etwa 100°C. Dies sind beispielsweise C₄-C₁₀-Alkohole, wie etwa Butanol, Hexanol, 2-Ethylhexanol u.s.w.. Bevorzugt werden Hexanol oder 2-Ethylhexanol, besonders bevorzugt 2-Ethylhexanol eingesetzt.
Anschließend wird ein molarer Unterschuss bis zu einem molaren Überschuss an Hydrazinhydrat zugesetzt. Die Menge an eingesetztem Hydrazinhydrat liegt dabei in einem Bereich von 5%igem molaren Unterschuss bis zu einem 5%igem molaren Überschuss. Bevorzugt ist ein 3%iger molarer Unterschuss bis ein 1 %iger molarer Überschuss, besonders bevorzugt ist ein 1 %iger molarer Unterschuss bis zur äquimolaren Menge.
Nach Zusatz des Hydrazinhydrates wird das Reaktionsgemisch zur Bildung des Isatinhydrazons etwa 10 bis 120 Minuten, bevorzugt 30 bis 100 Minuten und besonders bevorzugt 40 bis 80 Minuten auf der Reaktionstemperatur gehalten. Die Reaktionstemperatur liegt dabei zwischen 15 und 185°C, bevorzugt zwischen 20 und 100°C und besonders bevorzugt zwischen 30 und 60°C.

Das gebildete Isatinhydrazon wird nicht aus dem Reaktionsgemisch isoliert, sondern direkt weiterverarbeitet. Dazu wird ein tertiäres Amin als Katalysator zugesetzt. Geeignete tertiäre Amine sind beispielsweise Diazabicydoverbindungen, wie etwa Diazabicyclooctan (DABCO) oder Diazabicycloundecen (DBU), oder Trialkylamine, wie etwa Ethyldiisopropylamin. Bevorzugt wird als Katalysator DABCO eingesetzt.
Der Katalysator wird bei dem erfindungsgemäßen Verfahren in einer Menge von 5 bis 40 Mol%, bezogen auf die Ausgangsverbindung Isatin, zugegeben. Bevorzugt ist eine Katalysatormenge zwischen 10 und 30 Mol%, besonders bevorzugt zwischen 15 und 25 Mol%.
Die Reaktionstemperatur für diesen Schritt liegt zwischen 100 und 185°C, bevorzugt zwischen 120 und 160°C und besonders bevorzugt zwischen 125 und 145°C.
Hierbei kommt es zur Stickstoffabspaltung. Gleichzeitig wird das bei der Hydrazonbildung entstehende Reaktionswasser unter Normaldruck abdestilliert und die Reaktionslösung wird bis zur vollständigen Umsetzung auf der Reaktionstemperatur gehalten.
Gegebenenfalls kann das Reaktionswasser bereits vor Zugabe des Katalysators, beispielsweise mittels azeotroper Destillation aus dem Reaktionsgemisch abgezogen werden.
Anschließend wird zur Isolierung des entsprechenden 2-Oxindols ein Teil des Lösungsmittels abdestilliert. Bevorzugt werden etwa 50 bis 90%, besonders bevorzugt etwa 60 bis 80% des Lösungsmittels aus dem Reaktionsgemisch entfernt. Die verbleibende Reaktionslösung wird zur Kristallisation des 2-Oxindols unter Rühren langsam bis auf eine Temperatur zwischen 0°C und Raumtemperatur abgekühlt.
Gegebenenfalls kann vor der Kristallisation noch ein Ether, wie etwa Methylter.butylether (MTBE) oder Diisopropylether (DIPE) zugesetzt werden. Die zugesetzte Menge kann dabei bis zur doppelten Menge des noch vorhandenen Lösungsmittel betragen.
Danach wird das Produkt abfiltriert, bevorzugt mit dem bei der Reaktion eingesetzten Lösungsmittel oder mit dem vor der Kristallisation zugesetzten Ether kalt gewaschen, und getrocknet.

Durch das erfindungsgemäße Verfahren werden 2-Oxindole in hohen Ausbeuten und hoher Reinheit erhalten, wobei keine aufwendigen Reinigungsschritte erforderlich sind.
Bevorzugt wird das erfindungsgemäße Verfahren zur Herstellung von unsubstituiertem 2-Oxindol eingesetzt, wobei 2-Oxindol mit einer Reinheit bis zu 99,9 GC Fl.% erhalten wird.

### Beispiel 1:

393,3 g Isatin (2,63 mol, Gehalt 98,2 %) wurden in einem 2 I Doppelmantelgefäß vorgelegt und 2000 ml 2-Ethylhexanol zugefügt. Unter Rühren (KPG, 250 rpm) wurden innerhalb von 15 Minuten 131 ml Hydrazinhydrat (135,2 g, 2,70 mol, Gehalt 100,6 %) zugesetzt, wobei sich die Temperatur von 23 °C auf 45 °C erhöhte und eine intensiv orange Suspensiongebildet wurde.
Das Reaktionsgemisch wurde weiter erhitzt und 60 Minuten zur Bildung des Isatinhydrazons (gelbe Suspension) auf 90 °C gehalten. Danach wurden 61,2 g DABCO (0,55 mol, 98%) zugegeben und die Reaktionslösung kontinuierlich bis auf 130 °C erwärmt, wobei bei ca. 100 °C die Stickstoffabspaltung einsetzte. Gleichzeitig wurde- das Wasser (Reaktionswasser+Hydratwasser, insgesamt 96,5 ml) ausgetragen. Dabei kam es zum Rückfluß und zum Schäumen der Reaktionsmischung.
Das Reaktionsgemisch wurde bis zur vollständigen Umsetzung (ca.4,5 h) auf 130 °C gehalten.
Dann wurden bei 90-95 °C 1500 ml 2-Ethylhexanol abdestilliert (20-30 mbar, enthielt 0,02 Gew.% Hydrazin) und die verbleibende Reaktionslösung wurde zum Kristallisieren des 2-Oxindols unter Rühren langsam auf Raumtemperatur abgekühlt (in 3 h von 130 °C auf 50 °C, danach über Nacht bei Raumtemperatur stehen gelassen).
Das Kristallisat wurde abfiltriert, mit 5 x 50 ml kaltem 2-Ethylhexanol (0°C) nachgewaschen und trocken gesaugt.
Es wurden 509 g Feuchtprodukt erhalten, das ca. 48 Stunden im Vakuumtrockenschrank (80-90 °C und 50-65 mbar) bis zur Gewichtskonstanz getrocknet wurde. Insgesamt wurden 295 g 2-Oxindol Trockenprodukt (hellbraun) erhalten, das entsprach 84,4 % Gesamtausbeute.
Das Trockenprodukt wurde anschließend in der Reibschale zerkleinert und homogenisiert.

### Beispiel 2:

400,6 g Isatin (2,72 mol, Gehalt 98,2 %) wurden in einem 2 l Doppelmantelgefäßvorgelegt und 2000 ml 2-Ethylhexanol zugefügt. Unter Rühren (KPG, 250 rpm) wurden innerhalb von 15 Minuten 131 ml Hydrazinhydrat (135,2 g, 2,70 mol, Gehalt 100,6 %) zugesetzt, wobei sich die Temperatur von 23 °C auf 45 °C erhöhte und sich eine intensiv orange Suspension bildete.
Das Reaktionsgemisch wurde weiter erhitzt und 60 Minuten zur Bildung des Isatinhydrazons (gelbe Suspension) auf 50 °C gehalten. Danach wurden 61,2 g DABCO (0,55 mol, 98%) zugegeben und die Reaktionslösung kontinuierlich bis auf 130 °C erwärmt, wobei bei ca. 100 °C die Stickstoffabspaltung einsetzte. Gleichzeitig wurde das Wasser (Reaktionswasser+Hydratwasser+ 2-Ethylhexanol, insgesamt 114,7 g) ausgetragen. Dabei kam es zum Rückfluß und zum Schäumen der Reaktionsmischung.
Das Reaktionsgemisch wurde bis zur vollständigen Umsetzung (5 h) auf 130 °C gehalten.
Dann wurden bei 90-95 °C 1500 ml 2-Ethylhexanol abdestilliert (20-30 mbar, enthielt 0,02 Gew.% Hydrazin), die Reaktionslösung wurde auf 50 °C abgekühlt und mit 732 g MTBE versetzt und anschließend zum Kristallisieren des 2-Oxindols unter Rühren langsam auf 5 °C gekühlt (in 3 h von 130 °C auf 50 °C, danach über Nacht bei Raumtemperatur stehen gelassen).
Das Kristallisat wurde abfiltriert, mit 3 x 50 ml kaltem MTBE (5°C) nachgewaschen und trocken gesaugt.
Es wurden 328,1 g Feuchtprodukt erhalten, das ca. 48 Stunden im Vakuumtrockenschrank (80-90 °C und 50-65 mbar) bis zur Gewichtskonstanz getrocknet wurde. Insgesamt wurden 298,7 g 2-Oxindol Trockenprodukt (hellbraun) erhalten, das entsprach 83,1 % Gesamtausbeute.

### Beispiel 3:

40,39 g Isatin (0,27 mol, Gehalt 98,2 %) wurden in einem 500 ml Vierhalskolben vorgelegt und 200 ml 2-Ethylhexanol zugefügt. Unter Rühren (KPG, 250 rpm) wurden innerhalb von 15 Minuten 13,1 ml Hydrazinhydrat (13,5 g, 0,27 mol, Gehalt 100,6 %) zugesetzt, wobei sich die Temperatur von 23 °C auf 45 °C erhöhte und sich eine intensiv orange Suspension bildete.
Das Reaktionsgemisch wurde weiter erhitzt und 60 Minuten zur Bildung des Isatinhydrazons (gelbe Suspension) auf 50 °C gehalten. Danach wurden 6,12 g DABCO (55 mmol, 98%) zugegeben und das Reaktionsgemisch noch ca. 0,5 h bis zur Lösung des DABCO erwärmt.
Anschließend wurde die Suspension über einen Tropftrichter in einen Vorlagekolben mit 35 ml 2-Ethylhexanol, welches zuvor auf 145-150 °C vorgeheizt wurde, portionsweise chargiert. Da bei Zugabe der kälteren Suspension zum vorgeheizten 2- Ethylhexanol ein sofortiger Temperaturabfall eintrat, der die Stickstoffabspaltung verlangsamt, wurde, um dies zu vermeiden, die Isatinhydrazon-Suspension in Portionen von jeweils ca. 5 ml im Abstand von 2-3 Min. zugegeben, so dass die Temperatur in der Vorlage im Zielbereich von 140 -145 °C gehalten werden konnte. Gelegentlich wurde auch eine Verlängerung des Taktes auf bis zu 5 Min. erforderlich.
Während der Dosierung (1,5-2 h) schäumte die Reaktionssuspension stark, insbesondere am Ende der Reaktion.
Während der Stickstoffabspaltung wurden ca. 6,5 g eines zweiphasigen Gemisches aus Wasser und etwas 2-Ethylhexanol abdestilliert. In diesem Destillat 1 wurde kein Hydrazin gefunden.
Das Reaktionsgemisch wurde bis zur vollständigen Umsetzung (noch ca.1,5 Stunden) auf 140-145 °C gehalten (IPC-Kontrolle, GC: vollständiger Verbrauch des Hydrazons 0,1-0,2 Fl. %).
Dann wurden bei 90-95 °C 185 ml 2-Ethylhexanol abdestilliert (20-30 mbar, enthielt 0,02 Gew.% Hydrazin). Die Reaktionslösung wurde auf 55 °C heruntergekühlt und 80 g MTBE zugesetzt. Die Reaktionslösung wurde zum Kristallisieren des 2-Oxindols unter Rühren auf 5 °C gekühlt (in 0,5-1 h, danach über Nacht bei RT stehen gelassen).
Das Kristallisat wurde abfiltriert, mit 3 x 5 ml kaltern MTBE (0°C) nachgewaschen und trocken gesaugt.
Es wurden 30,0 g Feuchtprodukt erhalten, das ca. 48 Stunden im Vakuumtrockenschrank (80-90 °C und 50-65 mbar) bis zur Gewichtskonstanz getrocknet wurde. Insgesamt wurden 23,9 g 2-Oxindol Trockenprodukt (hellbraun) erhalten, das entsprach 66,5 % Gesamtausbeute.

| Gehalt GC: | |
|---|---|
| 2-Oxindol | 99,8-99,9 Fl. % |
| Verunreinigungen | < 0,1 Fl.% |

### Beispiel 4:

14,8 g Isatin (99 mmol, Gehalt 98,2 %) wurden in einem 250 ml Dreihalskolben vorgelegt und 150 ml 2-Ethylhexanol zugefügt. Unter Rühren wurden 6,0 g Hydrazinhydrat (117 mmol, Gehalt 98 %) zugesetzt.
Das Reaktionsgemisch wurde 15 Min. zur Bildung des Isatinhydrazons (gelbe Suspension) auf 90 °C erhitzt. Reaktionswasser wurde bei 90 °C/100 mbar azeotrop mit 1-Hexanol entfernt.
Danach wurden 2,4 g DABCO (21 mmol, 98%) zugegeben und die Suspension 2,5 h auf 130 °C erwärmt. Nach Ende der Reaktion (GC-Kontrolle: Hydrazon 0,3 Fl.%) wurden 100 ml 1-Hexanol abdestilliert (80 °C/50 mbar). Die verbleibende Lösung wurde auf 5 °C gekühlt, der abgeschiedene Feststoff abfiltriert und im Vakuumtrokkenschrank bei 70 °C getrocknet.
Insgesamt wurden 8,0 g 2-Oxindol Trockenprodukt (hellbraun) erhalten, das entsprach 66,5 % Gesamtausbeute. Reinheit: 99,9 Fl.%

## Patentansprüche

1. Verfahren zur Herstellung von 2-Oxindolen der Formel (II) 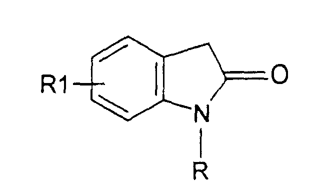 in der R H, CH₃, Phenyl oder Benzyl und R₁ H, C₁-C₄-Alkyl, C₁-C₄-Alkoxy, Phenyl, Phenoxy, Halogen, Amino, Nitro oder Hydroxy sein können, durch Umsetzung der korrespondierenden Isatinen der Formel (I) 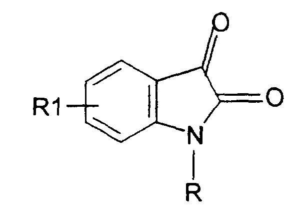 in einem polaren Lösungsmittel mit Hydrazinhydrat in Gegenwart eines tertiären Amins als Katalysator, **dadurch gekennzeichnet, dass** das Isatin bei einer Temperatur von 15 bis 185°C zu dem entsprechenden Isatinhydrazon umgesetzt wird, das direkt durch Zusatz von Diazabicyclooctan und/oder Diazabicycloundecan und/oder Ethyldiisopropylamin als Katalysator bei Temperaturen von 100 bis 185°C unter Abdestillieren des gebildeten Reaktionswassers zu dem entsprechenden 2-Oxindol der Formel weiterreagiert, welches dann durch Abdestillieren des Lösungsmittels und Kristallisieren aus dem Reaktionsgemisch isoliert wird.

2. Verfahren nach Anspruch 1, **dadurch gekennzeichnet, dass** Isatin der Formel (I) mit R gleich H und R₁ H oder OCH₃ zu dem korrespondierenden 2-Oxindol der Formel (II) umgesetzt wird.

3. Verfahren nach Anspruch 1, **dadurch gekennzeichnet, dass** als polares Lösungsmittel Hexanol oder 2-Ethylhexanol eingesetzt werden.

4. Verfahren nach Anspruch 1, **dadurch gekennzeichnet, dass** Hydrazinhydrat in 5%igem molaren Unterschuss bis 5%igem molaren Überschuss, bezogen auf das Isatin der Formel (I), zugesetzt wird.

5. Verfahren nach Anspruch 1, **dadurch gekennzeichnet, dass** die Reaktionstemperatur zur Bildung des Isatinhydrazons zwischen 20 und 100°C liegt.

6. Verfahren nach Anspruch 1, **dadurch gekennzeichnet, dass** das tertiäre Amin in einer Menge von 5 bis 40 Mol%, bezogen auf das Isatin der Formel (I), eingesetzt wird.

7. Verfahren nach Anspruch 1, **dadurch gekennzeichnet, dass** das Reaktionswasser bereits vor der Zugabe des Katalysators durch azeotrope Destillation aus dem Reaktionsgemisch entfernt wird.

8. Verfahren nach Anspruch 1, **dadurch gekennzeichnet, dass** zur Isolierung des 2-Oxindols der Formel (II) 50 bis 90% des Lösungsmittels abdestilliert werden und die verbleibende Reaktionslösung zur Kristallisation des 2-Oxindols der Formel (II) abgekühlt wird, worauf dieses abfiltriert, gewaschen und getrocknet wird.

9. Verfahren nach Anspruch 8, **dadurch gekennzeichnet, dass** dem Reaktionsgemisch vor der Kristallisation ein Ether zugesetzt wird.

## Claims

1. A process for preparing 2-oxindoles of the formula (II) 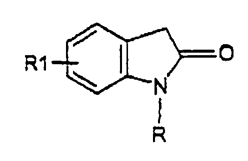 where R is H, CH₃, phenyl or benzyl, R₁ is H, C₁-C₄-alkyl, C₁-C₄-alkoxy, phenyl, phenoxy, halogen, amino, nitro or hydroxy, by reacting the corresponding isatins of the formula (I) 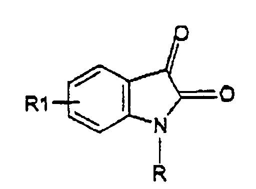 in a polar solvent with hydrazine hydrate in the presence of a tertiary amine catalyst, **characterized in that** the isatin is converted to the corresponding isatin hydrazone at a temperature of from 15 to 185°C which is directly reacted further by adding a diazabicyclooctane and/or diazabicycloundecane and/or ethyldiisopropylamine catalyst at temperatures of from 100 to 185°C while distilling off the water of reaction formed to give the corresponding 2-oxindole of the formula which is then isolated by distilling off the solvent and crystallizing out of the reaction mixture.

2. The process as claimed in claim 1, **characterized in that** the isatin of the formula (I) where R is H and R₁ is H or OCH₃ is converted to the corresponding 2-oxindole of the formula (II).

3. The process as claimed in claim 1, **characterized in that** the polar solvent used is hexanol or 2-ethylhexanol.

4. The process as claimed in claim 1, **characterized in that** hydrazine hydrate is added in from a 5% molar deficiency to a 5% molar excess, based on the isatin of the formula (I).

5. The process as claimed in claim 1, **characterized in that** the reaction temperature for forming the isatin hydrazone is from 20 to 100°C.

6. The process as claimed in claim 1, **characterized in that** the tertiary amine is used in a quantity of from 5 to 40 mol% based on the isatin of the formula (I).

7. The process as claimed in claim 1, **characterized in that** the water of reaction is already removed from the reaction mixture by azeotropic distillation before the catalyst is added.

8. The process as claimed in claim 1, **characterized in that** the 2-oxindole of the formula (II) is isolated by distilling off from 50 to 90% of the solvent and cooling the remaining reaction solution to crystallize the 2-oxindole of the formula (II) which is then filtered off, washed and dried.

9. The process as claimed in claim 8, **characterized in that** an ether is added to the reaction mixture before crystallization.

## Revendications

1. Procédé de fabrication de 2-oxindoles répondant à la formule (II) 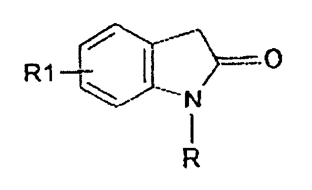 dans laquelle R peut représenter un atome de H, un groupe CH₃, un groupe phényle ou un groupe benzyle et R₁ peut représenter un atome de H, un groupe alkyle en C₁-C₄, un groupe alcoxy en C₁-C₄-, un groupe phényle, un groupe phénoxy, un atome d'halogène, un groupe amino, un groupe nitro ou un groupe hydroxy, par transformation des isatines correspondantes répondant à la formule (I) 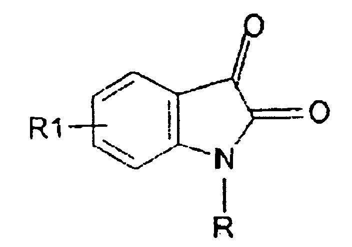 dans un solvant polaire avec de l'hydrate d'hydrazine en présence d'une amine tertiaire comme catalyseur, **caractérisé en ce que** l'isatine est transformée à une température de 15 à 185 °C en hydrazone d'isatine correspondant , lequel réagit à son tour directement par ajout de diazabicyclooctane et/ou de diazabicycloundécane et/ou d'éthyldiisopropylamine comme catalyseur à une température de 100 à 185 °C après élimination de l'eau de réaction formée par distillation et forme le 2-oxindole correspondant de la formule, lequel est ensuite isolé du mélange de réaction par élimination du solvant par distillation et cristallisation.

2. Procédé selon la revendication 1, **caractérisé en ce que** l'isatine de formule (I) avec R représentant H et R₁ représentant un atome d'H ou un groupe OCH₃ est transformée en 2-oxindole correspondant de formule (II).

3. Procédé selon la revendication 1, **caractérisé en ce que** l'on utilise comme solvant polaire de l'hexanol ou de 2-éthylhexanol.

4. Procédé selon la revendication 1, **caractérisé en ce que** on ajoute de l'hydrate d'hydrazine en déficit molaire de 5 % jusqu'à un excédent molaire de 5 %, rapporté à l'isatine de formule (I).

5. Procédé selon la revendication 1, **caractérisé en ce que** la température de réaction pour la formation de hydrazone d'isatine se situe entre 20 et 100 °C.

6. Procédé selon la revendication 1, **caractérisé en ce que** l'amine tertiaire est utilisée en une quantité de 5 à 40 mol%, rapporté à l'isatine de formule (I).

7. Procédé selon la revendication 1, **caractérisé en ce que** l'eau de réaction est déjà éliminée du mélange de réaction avant l'ajout du catalyseur par distillation azéotropique.

8. Procédé selon la revendication 1, **caractérisé en ce que** 50 à 90 % du solvant sont éliminés par distillation pour isoler le 2-oxindol de formule (II), et **en ce que** la solution de réaction restante est refroidie pour cristalliser le 2-oxindol de formule (II), lequel est ensuite filtré, lavé et séché.

9. Procédé selon la revendication 8, **caractérisé en ce qu'**un éther est ajouté au mélange de réaction avant la cristallisation.
